(19) Europäisches Patentamt
European Patent Office
Office européen des brevets

(11) Publication number: **0 052 999**
**B1**

(12) **EUROPEAN PATENT SPECIFICATION**

(45) Date of publication of patent specification: **16.01.85**

(51) Int. Cl.[4]: **C 07 C 31/125,** C 07 C 29/14, C 07 C 29/16, C 07 C 45/61

(21) Application number: **81305441.8**

(22) Date of filing: **18.11.81**

(54) **Preparation of plasticizer alcohols from propylene-butene mixtures.**

(30) Priority: **20.11.80 US 208726**

(43) Date of publication of application:
**02.06.82 Bulletin 82/22**

(45) Publication of the grant of the patent:
**16.01.85 Bulletin 85/03**

(84) Designated Contracting States:
**BE DE FR GB IT NL**

(56) References cited:
**DE-B-1 024 943**
**DE-B-1 157 593**
**GB-A-1 014 543**
**GB-A-1 183 637**
**GB-A-1 252 678**
**US-A-3 766 279**

**F. Usinger, Mono-olefins chemistry and technology (1968), p. 816-817**

(73) Proprietor: **MONSANTO COMPANY**
**Patent Department 800 North Lindbergh Boulevard**
**St. Louis, Missouri 63166 (US)**

(72) Inventor: **Forster, Denis**
**32 Woodcrest Drive**
**Ladue Missouri 63124 (US)**
Inventor: **Barker, George Edward**
**1616 Redgate Lane**
**St. Louis Missouri 63141 (US)**
Inventor: **Farrar, Martin Wilbur**
**858 Briarfarm Lane**
**Kirkwood Missouri 63122 (US)**

(74) Representative: **Lunt, John Cooper et al**
**Monsanto Europe S.A. Patent Department**
**Avenue de Tervuren 270-272 Letter Box No 1**
**B-1150 Brussels (BE)**

**EP 0 052 999 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention is concerned with a method for preparing plasticizer alcohols from mixtures of propylene and butenes in an overall selective process.

### Background of the invention

Various lower olefinic feedstocks are available from petroleum sources, and many procedures are known for converting lower olefins to higher olefins, or to higher molecular weight compounds with various functional groups. Among such procedures are catalytic dimerization procedures for converting butenes to octenes, and such octenes can be catalytically hydroformylated to aldehydes, which can be reduced to alcohols. Lower olefins can be hydroformulated, using rhodium, cobalt, or other catalysts, to the corresponding aldehydes. Aldehydes in turn can be converted to higher aldehydes by the well known aldol reaction, such as that taught in U.S. Patent 2,921,089 for converting n-valeraldehyde to 2-propyl-2-heptenal, and then hydrogenated to 2-propylheptanal and 2-propylheptanol. It is also known that various alcohols can be utilized to esterify phthalic acid to form useful plasticizers, e.g. 2-ethylhexanol, 2-propylheptyl alcohol, decyl alcohols, etc. as disclosed in the aforesaid 2,921,089. It is also known to conduct an aldol reaction of mixtures of normal and branched aldehydes under conditions which force cross aldol reaction of the branched aldehyde with the normal aldehyde, see U.S. Patent 2,852,563.

Nine-carbon alcohols are extensively used in plasticizers such as diisononyl phthalate. Such alcohols are commercially produced by hydroformulation of a branched eight-carbon olefin obtained by the dimerization of butenes over an acid catalyst. This approach has the disadvantage of poor selectivity. The dimerization reaction gives a highly branched product, and the hydroformulation of the branched eight-carbon olefins requires very strenuous reaction conditions and the selectivity is much poorer than that with comparable straight chain olefins. The dimerization also produces higher oligomers which are not suitable for conversion to plasticizer alcohols, but must find alternate uses, such as in gasoline.

The present invention provides a process for preparing plasticizer alcohols which comprises subjecting a mixture of aldehydes to an aldol condensation reaction and hydrogenating the condensation products to saturated alcohols, characterised in that the mixture of aldehydes is a mixture of butyl and amyl aldehydes obtained by conducting an oxo reaction on a mixture of propylene and butenes in a ratio of 2:1 to 1:3 on a molar basis.

The aldehyde intermediates contain some branched isomers, but the aldol reaction can be conducted so as to lessen participation of the branched isomers therein, particularly reaction of 2-methyl-butanal. The use of both propylene and butene as feedstock permits production of a product having the combined properties resulting from use of these two precursors, particularly with regard to volatility, softening ability and plasticizer efficiency. The alcohols produced in the process for the most part have $C_8$ to $C_{10}$ carbon chains, with the average usually being close to 9 carbon atoms, depending however upon the ratio in which propylene and butenes are utilized. The alcohols produced partially or entirely from propylene feedstock can compensate for some of the shortcomings of product from butene feedstock or particular aldehyde isomers produced therefrom.

The hydroformulation in the present process can be conducted under mild conditions because propylene and butenes are highly reactive, and with good selectivity. The conversion of the short chain aldehydes to the plasticizer range chains is accomplished with a highly selective aldol reaction. The resulting higher aldehydes are readily hydrogenated to alcohols, providing an overall process with very good selectivity to the desired plasticizer range alcohols. For plasticizer alcohols, it is generally desirable to limit the degree of chain branching as some types of branching have an adverse effect upon properties. In the present process the aldol reaction can be conducted with a mixture of aldehydes so as to lessen or minimize product produced from one or more of the branched aldehydes therein, thereby lessening the branching in the product. In one particular aspect, the use of propylene along with butenes feedstock counteracts some of the deleterious property resulting from product from 3-methyl-butanal, making it feasible to use mixed butene feedstock with some isobutylene, and still obtain acceptable plasticizer properties.

### Detailed description of the invention

In the production of plasticizer alcohols from olefins, typical procedures introduce branching into the alcohol product. The branching has a significant effect upon properties when the alcohols are utilized as plasticizers in the form of phthalate esters. The aldol reaction utilized herein in itself results in a branched product. However, additional branching is introduced if branched aldehydes are caused to react in the aldol reaction, and it is desirable to control the amount of such multi-branched product. One possible approach is to use only normal aldehydes in the aldol reaction, but this requires extensive distillation or other procedures to obtain aldehydes of principally normal structure, when the aldehydes are a mixture of branched and normal aldehydes obtained in a hydroformulation.

In the hydroformulation of propylene and butenes, it is possible by use of moderate conditions to obtain a fairly high ratio of normal to branched aldehydes of at least 2.8:1 e.g. about 3:1. Such mixtures

can be obtained by hydroformylating either propylene or linear butenes, or mixtures of propylene and linear butenes. The propylene to butenes mole ratio can, for example be in the range 1:1 to 1:2. Mixtures of $C_4$ and $C_5$ aldehydes, containing branched along with normal aldehydes, can then be utilized in an aldol reaction to obtain higher aldehydes with a higher proportion of the normal than branched aldehydes taking part in the reaction and forming moieties in the product. The $C_4$ and $C_5$ normal aldehydes both react at good rates in the aldol reaction to produce $C_8$, $C_9$ and $C_{10}$ enals. However some of the branched aldehydes, particularly 2-methylbutanal, react at relatively slow rates, and the aldol reaction can be conducted so as to leave some of the branched aldehydes unreacted, thereby increasing the amount of product from normal aldehyde. In effecting the aldol reaction, it is preferred to use components and conditions such that 70% to 90% by weight of the product is from normal aldehydes. The aldol reaction can be represented:

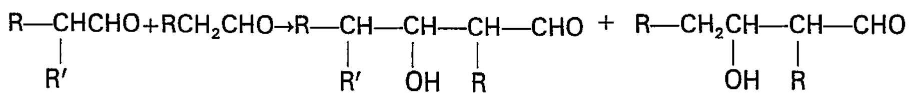

*A* *B* *C* *D*

in which the relative amounts of *C* and *D* depend upon the particular aldehydes *A* and *B* and their amounts, as well as reaction conditions. It will be noted that the illustration shows product with the carbonyl carbon of the branched aldehyde joined to the $\alpha$-carbon of the normal aldehyde, as this is the usual way in which such components react. Moreover, with the aldehydes involved in the present process, there is little self condensation of the branched aldehyde illustrated by *A*. When the branched aldehyde takes part in the reaction, either by self condensation or by reaction with *B*, it introduces additional branching into the product over that which is present from the aldol reaction of two normal aldehydes, and such product can be termed multi-branched product, in comparison to the single-branched product of normal aldehydes. Where references are made herein to the amounts of product produced from normal aldehyde, such multi-branched product will not be counted as it is partially produced from branched aldehyde and has the resulting characteristics.

In the present process, *A* and *B* can each be mixtures of 4-carbon and 5-carbon aldehydes and this presents additional possibilities for condensation in various combinations to produce $C_8$, $C_9$ and $C_{10}$ products. In general the four- and five-carbon aldehydes are both reacted to fairly good conversions. The aldol reaction may be conducted for a time and under conditions sufficient to convert upwards of 90 to 95% of the normal aldehydes to higher enal or other condensation product. More specifically, the aldol reaction can be conducted so as to convert more than 90% of the normal aldehydes from the oxo reaction to higher aldehydes, but leaving most of the 2-methylbutanol from the oxo reaction unreacted.

While the products illustrated in the reactions above are the primary aldol product, they are generally quickly dehydrated to an enal, as illustrated:

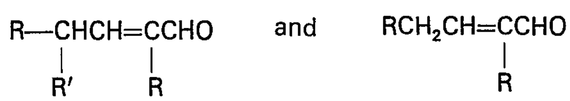

The enals can readily be hydrogenated in one or two stages to form the corresponding saturated alcohols.

In the present process the propylene and butenes can be used effectively and efficiently as a mixture in the hydroformulation reaction, and the resulting aldehyde product mixture can be utilized effectively in an aldol reaction to obtain products suitable for reduction to plasticizer alcohols. In a modification of the process, the hydroformylation reaction is conducted with separate propylene and butenes streams and the reaction products are combined. The properties resulting from propylene and butenes are to some extent complementary and combinations can give a desirable blend of properties, presenting an advantage in addition to the efficiency of the process. The content from propylene and butenes in the plasticizer alcohol will generally be in a ratio from 2 propylene:1 butenes to 1 propylene:3 butenes on a mol basis, with up to 20% of the butenes being isobutene. Higher amounts of isobutene can be used, and amounts of isobutene up to 50% of the content may be found in butenes from refinery streams, and conveniently used. However, the 3-methylbutanal resulting from hydroformulation of isobutene reacts well in the aldol reaction and the resulting di-branched product has an adverse effect upon plasticizer properties. Consequently there is advantage in using linear butenes, or in keeping isobutene content relatively low. The use of mixtures of components from propylene and butenes makes it feasible to use a higher isobutene content in the butenes than might otherwise be acceptable. Thus a plasticizer alcohol produced entirely from butenes with 50% isobutene content would lead to very poor plasticizer properties, with a $T_f$ near −30° or so. Use of propylene in conjunction with the butenes would improve the $T_f$ value.

In the description immediately above and elsewhere herein there may be reference to content from propylene, butenes, etc. It will be recognized that the reference is ordinarily to the source of the

material, and not to its state in a particular product. Thus the propylene in materials produced therefrom will be in the form of 4-carbon aldehydes and 8- and 9-carbon aldehydes and alcohols, while the butenes are in the form of 5-carbon aldehydes and 9- and 10-carbon aldehydes and alcohols. The alcohols in turn are in the form of phthalate esters when evaluated for use in plasticization.

With further regard to plasticizer properties, the contribution from butenes lowers volatility of the plasticizer, and that from propylene gives improved Shore Hardness properties, making the values somewhat better than that from butenes alone.

A general procedure suitable for carrying out the aldol reactions and converting the product to alcohols can be described as follows. The mixture of aldehydes is slowly added to vigorously stirred aqueous caustic (1 molar) at 90—95°C. The weight ratio of the aldehydes to aqueous phase is 2:1. The reaction is monitored by gas chromatographic analysis, and continued until n-pentanal conversion exceeds 95% (1 to 2 hours), at which time there are still substantial amounts of unconverted branched aldehydes. The organic phase is separated and hydrogenated without further purification. Hydrogenation can conveniently be effected with a cobalt on kieselguhr catalyst at 160°C and 10436 KPa of $H_2$. The resulting alcohols are distilled, removing some $C_4$ and $C_5$ alcohols at atmospheric pressure, and continuing the distillation at 1333 Pa.

The aldol reaction can be carried out utilizing the usual aldol catalysts and conditions to promote the aldol reaction, using elevated temperatures upwards of 60°C., particularly temperatures of 90°C to 130°C or possibly up to 150°C or higher, if desired. The reaction is operable over broad pressure ranges including pressures less than atmospheric as well as elevated pressures, but will usually be effected at slightly elevated pressures sufficient to maintain the reactants substantially in the liquid state. The reaction can also conveniently be conducted at reflux.

The aldol reaction can utilize strongly alkaline catalyst, such as sodium and potassium hydroxide, or sodium and potassium cyanide. The concentration of the aqueous alkali can be varied, but molar or similar concentrations can be used, and concentrations selected will generally be in the range of 1 to 10% by weight. Alkali hydroxide can conveniently be used in such amounts. The amount of aqueous alkali to aldehyde reactant can also vary, for example, from 15% by volume aqueous alkali up to 75% by volume aqueous alkali. The aldol reaction will be run for a sufficient time to obtain the desired degree of conversion, which for batch reactions may be in the range of 1 to 3 hours, while in continuous reaction times of less than 5 minutes are achievable. The reaction is stopped by permitting the reaction mixture to cool and separating the organic reaction phase from the aqueous alkali phase. Since the n-pentanal reacts more rapidly than its isomers, the proportion of the isomers in the unreacted aldehyde increase and it therefore is not generally desirable to separate and recycle unreacted components to the reaction.

It is important that the hydroformylation of both propylene and mixed linear butenes gives a relatively high ratio of normal aldehydes, as this contributes to the feasibility of using the aldehyde mixture for an aldol reaction to obtain a fairly high yield of monobranched enals from normal aldehydes. The use of moderate temperatures in the hydroformylation contributes to obtaining about a 3:1 mixture of normal to branched aldehyde, i.e. a mixture in which both the butyl and amyl aldehydes are in approximate 3:1 ratio of normal to branched aldehydes. Thus temperatures sufficient to produce an appreciable reaction rate, ranging from 80° to 100°C, or so can be used and temperatures on up to 125—130°C can be employed to obtain better reaction rates. Still higher temperatures up to 150°C or higher can be used but with a tendency to produce more branched aldehyde than desired. To some extent high catalyst concentrations can be employed to obtain desired reaction rates, even at relatively low temperatures. Cobalt catalyst is especially suited to obtain the desired high proportion of normal aldehyde. Unmodified cobalt carbonyl catalyst can conveniently be used. Such catalyst, conventionally designated as cobalt octacarbonyl, can be provided or employed in many forms known to be useful as a hydroformylation catalyst, although it may be necessary to exercise some choice to provide catalyst best suited to obtaining a high proportion of normal product.

The oxo stage of the reaction can be conducted under the usual conditions pertaining to cobalt catalyzed hydroformylation reactions, with attention to the temperature conditions as described above. Usual pressure conditions apply, such as 6900—27,600 or up to 34,500 KPa total pressure, with most of the pressure being from the carbon monoxide and hydrogen supplied. The carbon monoxide and hydrogen are conveniently used in 1:1 ratio and obtained from usual synthesis gas sources, but other ratios can be employed in keeping with known hydroformylation practice. The reaction can be carried to the desired stage of completion in 1 to 3 hours or so on a batch basis, varying with time, temperature, pressure and catalyst concentration. Pressures should be sufficient to provide catalyst stability, but there is generally no reason to exceed 35,000 KPa. With rhodium catalyst, preferred temperatures and pressure conditions are generally milder than with cobalt.

The oxo reaction can be conveniently conducted either without a solvent or with solvents and, employing concentrations customary for homogeneous catalyzed reactions, such as 2 to 10 molar or greater concentrations of the olefins in a solvent, e.g., hydrocarbon solvents such as toluene, and 0.1% to 1% by weight, based on cobalt, of catalyst.

It is possible to obtain butene streams which are substantially linear and substantially free of isobutylene, in which no more than 2% or so of the butenes is isobutylene, and it is advantageous to

use such butene streams. However, somewhat more isobutene can be tolerated, so long as the total of the resulting 3-methylbutanal together with other branched aldehydes in the aldehydes to be reacted does not become undesirably high. The 3-methylbutanal is more prone to react in the aldol reaction than the 2-methylbutanal isomer.

The hydrogenation of the enals from the aldol reaction can be conducted under the usual catalytic hydrogenation conditions for reducing olefinic bonds and aldehyde groups. The carbon-to-carbon bond reduces more rapidly and at a lower temperature than the aldehyde group, e.g. at about 90°C, with cobalt on Kieselguhr catalyst at elevated hydrogen pressure. The hydrogenation will generally be carried out at 3450—13,800 KPa, or greater hydrogen pressures and temperatures of 130° to 200°C or higher, although any temperatures which are effective with a particular catalyst can be used. The stated conditions will be effective for reducing both the carbon-to-carbon bond and the aldehyde group to obtain saturated alcohol. Various other hydrogenation catalysts can be used including platinum and platinum on carbon catalysts, copper chromite, activated nickel etc., and individual catalysts can be utilized in conjunction with other catalysts.

The present invention involves an oxo reaction, followed by an aldol reaction, and then a hydrogenation to convert enals to alcohols. For large scale operations, the oxo reaction will be conducted with usual provisions for separating gaseous reactants and products, and catalyst, from the aldehyde products, with recycle as appropriate. The aldehyde product mixture will then be subjected to an aldol reaction, followed by decantation and water washing or other simple procedures to separate the organic product-containing phase from the aqueous phase. The product phase is then hydrogenated, converting both unreacted lower aldehydes and product enals to the corresponding alcohols. The hydrogenation is followed by a distillation to remove light ends, followed by a distillation to remove $C_4$ and $C_5$ alcohols, and then a distillation of recovered plasticizer alcohols. Both the lower and plasticizer alcohols can then be treated in further hydrogenation polishing operations to improve the alcohol quality by insuring complete hydrogenation.

As an alternative to the above procedure, it is possible to separate the unreacted lower aldehydes by distillation from the product enals prior to hydrogenation. For convenience of separation, distillation of the alcohols is generally preferred, and then the four- and five-carbon components are in the form of alcohols. However, if the aldehydes are desired for some purpose, separation is appropriate, and this has the advantage of avoiding unnecessary hydrogen use.

Hydroformylation of propylene with a cobalt carbonyl catalyst at 120°C and 20,700 KPa $CO/H_2$ gives an isomeric ratio of n-butanal to 2-methyl-propanal of 3:1. Hydroformylation of n-butenes containing 50% 1-butene also produces a linear to branched ratio of 3:1 at the stated reaction conditions. The following example illustrates the aldol reaction of a 1:1 mole mixture of $C_4$ and $C_5$-aldehydes with the linear to branched ratio being 3:1 in both cases.

Example 1

A 1 molar aqueous KOH solution, 500 ml, was placed in a reaction vessel. A mixture of aldehydes containing 655 grams n-pentanal, 217 grams 2-methylbutanal, 548 grams butanal, and 184 grams isobutanal was placed in an addition funnel. The aqueous solution was heated to 85°C at reflux and addition of the aldehydes was begun and continued for about 90 minutes. Samples were taken periodically. After addition was completed, the reaction mixture was heated to reflux for an additional hour, and a final temperature of 90°C. The reaction mixture was cooled and transferred to a separatory funnel, where the lower phase was removed, leaving an organic phase of 1372 grams. The product, containing $C_8$, $C_9$ and $C_{10}$ unsaturated aldehydes, as well as substantial quantities of unreacted lower aldehydes, was hydrogenated with a cobalt on Kieselguhr catalyst at 160°C and 10,430 KPa.

The hydrogenation product was transferred to a still and distillation was commenced at atmospheric pressure with removal of low boiling material, primarily 2-methylbutanal, at head temperatures of 94—138°C. Distillation of the remainder was then conducted at 1333 Pa obtaining additional light fractions at head temperatures up to 82°C, and then the bulk of the product at temperatures of 82—114°C. This part of the product was used for preparation of a phthalate ester for plasticizer evaluation, as reported in Table 1.

In the product separation, a substantial amount of 2-methylbutanol was found, indicating that the 2-methylbutanal was relatively inactive in the aldol reaction, with apparently less than 50% of it reacting. All of the other components were reasonably reactive as shown by the relatively small amounts of other alcohols found in the product, although some isobutanol and lesser amounts of n-pentanol and n-butanol were found.

Example 2

Aldehydes were provided for an aldol reaction in the proportions to be expected from hydroformylation of a propylene and linear butene stream in ratio of 1 propylene:2 butene. The reaction was conducted generally as described in Example 1, using an aldehyde mixture containing 107.7 grams n-butanal, 34.8 grams isobutanal, 255.3 grams n-pentanal, and 85.1 grams 2-methylbutanal. The aldehydes were added to 144 ml aqueous KOH which had been heated to 92°C. The addition was completed in fifteen minutes. An additional 60 ml of the KOH solution was added

after twenty minutes reflux. The total reflux time was 3.5 hours. A 404 gram organic phase was separated from the reaction mixture. Hydrogenation was conducted as in Example 1.

Analysis of the hydrogenation product indicated that approximately 81% of the product was that resulting solely from normal aldehydes. The amount of 2-methylbutanol in the product showed that only about one-third of the 2-methylbutanal had reacted in the aldol reaction. The product was distilled, with some light materials being removed at atmospheric pressure and the distillation then continued at 1332 Pa with the bulk of the 8—10 carbon product being obtained in fractions at head temperatures of 89—108°C. A portion of the product from fractions at 98 to 108°C was blended with small amounts of the lower boiling 2-ethylhexanol and 2-ethyl-4-methyl-pentanol, giving a product about 82% from normal aldehydes, and used for preparation of a phthalate plasticizer. Evaluation of the plasticizer is reported in Table 1.

Example 3

Aldehydes were provided for an aldol reaction in portions expected from a 1:1 weight mixture of a propylene stream with a butenes stream which contains isobutylene along with linear butenes, the isobutylene constituting about 43% of the total. After hydroformylation the 3-methylbutanal from isobutylene is expected in approximately equivalent amount to n-pentanal. The aldehyde mixture contained 176.4 grams butanal, 59.5 grams isobutyraldehyde, 104.7 grams pentanal, 102.9 grams 3-methylbutanal and 35.2 grams 2-methylbutanal. The aldol reaction was conducted with 175 ml of aqueous 1 molar KOH with stirring at temperatures near 95°C for about 2.5 hours. The organic phase was hydrogenated under usual conditions.

The product was distilled, with some materials distilling at atmospheric pressure and distillation then being conducted at 1333 Pa, with fractions being taken at head temperatures of 78 to 85°C and then 85 to 105°, to obtain about 235 grams of distillate. The 78 to 85° fractions were subjected to redistillation at temperatures up to 70°C to remove a small portion of low-boiling material, and the residue was then combined with the higher boiling fractions and utilized to prepare phthalate ester for evaluation. From analysis it was concluded that the n-butanal, n-pentanal and 3-methylbutanal all reacted to nearly complete conversion in the aldol reaction, and that the isobutanal was more than 75% converted. The conversion of 2-methylbutanal was fairly low, less than 20%. The effective yield in the aldol reaction is around 91—93%. However, the fairly extensive branching in the product, much of it from product of 3-methylbutanal, has an adverse effect upon properties.

Plasticizer properties of phthalate ester in polyvinyl chloride (40%) are reported in Table 1, utilizing diisononyl phthalate and dioctylphthalate for comparison.

TABLE 1

| Plasticizer alcohol | | | | Volatility | |
|---|---|---|---|---|---|
| Source | Carbon No. average | $T_f$* | Shore hardness | 1 day % | 6 days % |
| Ex. 1 | 8.97 | –35.9 | 76 | 2.4 | 10.7 |
| Ex. 2 | 9.32 | –37.2 | 76 | 1.8 | 8.3 |
| Ex. 3 | 8.92 | –35.2 | 76 | 2.2 | 9.9 |
| Diisononyl phthalate | — | –36.0 | 76 | 1.8 | 7.0 |
| Dioctyl phthalate (2-ethylhexyl) | | –39.0 | 74 | 4.0 | 19.0 |

* The low temperature flexibility in degrees centigrade as determined in a standard Clash-Berg test.

Plasticizer alcohols in the form of phthalate esters must have a particular combination of plasticizer properties with respect to flexibility and hardness (softness) of the compositions plasticized therewith, and volatility. Thus it is desirable that the properties approach or exceed those of diisononyl phthalate, such as for plasticized polyvinyl chloride, a $T_f$ as low as –36°C, volatility as low as about 7% in a six day test, and Shore Hardness no greater than 76.

Example 4

In this example, a mixture of n-pentanal, 2-methylbutanal and n-butanal is employed in which the mole ratio of $C_5$ aldehydes to $C_4$ aldehyde is 1.7:1.

A mixture of n-butanal (264.05 gm), n-pentanal (401.7 gm) and 2-methylbutanal (134.3 gm) was added slowly (over about 2 1/2 hours) to a vigorously stirred sodium hydroxide solution (1970 ml, 0.8M) maintained at a temperature of about 94°C. After the addition was complete the heating was

continued for a further thirty minutes after which the reaction mixture was cooled to room temperature. The product consisted of two phases which were separated giving 704 gm of an organic phase. Gas chromatographic analysis of this phase showed that the n-butanal and n-pentanal had been reacted almost to completion but a substantial proportion of the 2-methylbutanal still remained. The organic phase was hydrogenated at 160°C and 10,430 KPa pressure of hydrogen in stirred autoclave in the presence of a cobalt on kieselguhr catalyst. The reaction was allowed to proceed for 1-1/4 hours. The reaction product was filtered and distilled at 1333 Pa. The fractions boiling in the range 88—98°C consisted of the eight, nine and ten carbon alcohols. The fractions were recombined to give a material representative of the $C_8$, $C_9$, $C_{10}$ ratios observed in the unfractionated material. This mixture of alcohols was converted to a phthalate ester and evaluated as a plasticizer for polyvinyl chloride as reported in Table 2.

Example 5

Aldol and hydrogenation reactions were carried out in accordance with the procedure of Example 4, but employing 170.7 gm n-pentanal, 57 gm 2-methylbutanal, and 72 gm n-butanal, for a $C_5$ aldehyde to $C_4$ aldehyde mole ratio of 2.65:1. The aldehydes were added to 700 ml of 0.8 M NaOH. The organic phase after the aldol reaction weighed 263 gm. Evaluation of the hydrogenation product is reported in Table 2.

Example 6

A mixture of 513.1 gm n-pentanal and 286.8 gm n-butanal was reacted by the procedure of Example 3. The $C_5$ aldehyde to $C_4$ aldehyde mole ratio was 1.5 to 1. The aldehyde mixture was added to a heated 1970 ml 0.8 M NaOH solution. The organic product layer weighed 698.4 gm. Evaluation of the hydrogenation product is reported in Table 2.

TABLE 2
Polyvinylchloride plasticizer properties

| Alcohol source | $T_f$ | Shore hardness | Volatility 1 day (%) | Volatility 6 days (%) |
|---|---|---|---|---|
| Ex. 4 | −37.6 | 76 | 1.7 | 9.4 |
| Ex. 5 | −38.4 | 77 | 1.5 | 7.0 |
| Ex. 6 | −39.5 | 75 | 1.7 | 8.5 |
| Diisononyl phthalate | −36.0 | 76 | 1.5 | 7.0 |

The present process is adaptable by minor changes in conditions or procedures to give desired relationship of product quality and process yield. In general, the higher the amount of multi-branched product, the higher the yield and the lower the by-product alcohol, and the lower the product quality. Conversely, the product quality is upgraded by limiting reaction of branched aldehydes, with some loss of yield to by-product alcohol. Even with such losses, the overall selectivity to desired plasticizer alcohols can still exceed that available by other means of converting alkenes to plasticizer alcohols. Thus with a $T_f$ of −34°C, very little by-product alcohol would be produced in the present process, while fairly high amounts would be produced to attain $T_f$ of −39 to −40°C. One simple variable to adjust is the water to aldehyde ratio in the aldol reaction. Raising the ratio increases the amount of multi-branched product, while reducing it decreases such product, all, of course, within the potential limits of such product from the amount of branched aldehyde in the reaction.

One optional procedure for improving the product involves removing a low boiling fraction by distillation following the oxo reaction. The material removed will be approximately 75% isobutanal and 25% n-butanal. This material can then by-pass the aldol step and be fed directly to the hydrogenation unit. This can improve the $T_f$ to the −38 to −39° range and give lower volatility. Alternatively, a multistage aldol reaction can be employed, and the low boiling fraction fed to the last stage, as, for example, the third stage of a three stage reaction. This would result in most of the n-butanal being completely reacted, but very little of the isobutanal as it is not introduced until most of the straight chain aldehydes have reacted. This procedure could give product resulting in $T_f$ of −37 to −38°C and volatility similar to that of diisononyl phthalate.

Additional approaches can take advantage of the low reactivity of 2-methylbutanal to lessen its participation in the aldol reaction. Thus the aldehydes from the oxo reaction can be divided, and one half reacted to high conversion in the aldol reaction, and unreacted aldehyde separated therefrom; this would be a relatively simple distillation of lower aldehydes from higher aldol products. The other

aldehyde half could then be added to the next stage of the aldol reaction. Such procedures could produce product to give $T_f$ in the −38 to −39° range, but with additional 2-methylbutanol and isobutanol by-product.

Some minor fractionation of the plasticizer alcohol product can also be employed to improve product quality. Thus isobutanal reacted in the aldol reaction appears principally in the product alcohol as 2-ethyl-4-methylpentanol, and this low boiling alcohol can be readily removed along with similarly low boiling materials. Removal of approximately 5% of this material from product can improve the $T_f$ of the phthalate of the product by about 0.5°C.

In addition to the various advantages discussed herein in use of a propylene and butene feedstock, the use of such combination permits a higher utilization of the feedstock olefin, i.e. better yield of product, with good product quality, than ordinarily obtainable by use of butenes only as feedstock.

## Claims

1. A process for preparing plasticizer alcohols which comprises subjecting a mixture of aldehydes to an aldol condensation reaction and hydrogenating the condensation products to saturated alcohols, characterised in that the mixture of aldehydes is a mixture of butyl and amyl aldehydes obtained by conducting an oxo reaction on a mixture of propylene and butenes, in a ratio of 2:1 to 1:3 on a molar basis.

2. A process of Claim 1 in which the aldol reaction is conducted so as to convert more than 90% of the normal aldehydes from the oxo reaction to higher aldehydes, but leaving most of the 2-methylbutanal from the oxo reaction unreacted.

3. A process of either of Claims 1 and 2 in which the oxo reaction is conducted with propylene and butenes feedstock and conditions so as to produce aldehydes with a normal to branched ratio of at least 2.8:1.

4. A process of any of Claims 1 to 3 in which up to 20% of the butenes is isobutylene.

5. A process of Claim 1 in which the ratios of the propylene and butenes are controlled together with the isobutylene content of the butenes and the aldol conditions so as to produce alcohols which are at least 70% derived from normal aldehydes.

6. A process of Claim 1 in which both the butyl and amyl aldehydes are in approximate 3:1 ratio of normal to branched aldehydes and the propylene to butenes mol ratio is in the range 1:1 to 1:2.

7. A process of Claim 1 modified in that the oxo reaction is conducted with separate propylene and butenes streams, and the reaction products are combined.

8. A process of any of Claims 1 to 7, in which at least a substantial portion of isobutanol from the oxo reaction of propylene is removed from the oxo product by distillation prior to conducting the aldol reaction.

9. A process of any of Claims 1 to 8 in which the oxo reaction is conducted at temperatures in the range 80° to 150°C and pressure sufficient to maintain catalyst stability but not over 34,500 KPa, and the aldol reaction is conducted in aqueous alkali of 1 to 10% concentration by weight.

10. A process of any of Claims 1 to 9 in which a substantial portion of the ethyl methyl pentanol produced in the process is separated from the other product alcohols by distillation in order to improve the product alcohol as a plasticizer alcohol.

11. A plasticizer obtained from plasticizer alcohols that have been prepared by a process according to any of Claims 1 to 10.

## Patentansprüche

1. Verfahren zur Herstellung von Weichmacher-Alkoholen, wobei ein Gemisch von Aldehyden einer Aldolkondensationsreaktion unterworfen wird und die Kondensationsprodukte zu gesättigten Alkoholen hydriert werden, dadurch gekennzeichnet, daß das Aldehydgemisch ein Gemisch von Butyl- und Amylaldehyden ist, das durch Durchführung einer Oxoreaktion mit einem Gemisch von Propylen und Butenen in einem molaren Verhältnis von 2:1 bis 1:3 erhalten wird.

2. Verfahren nach Anspruch 1, worin die Aldolreaktion so durchgeführt wird, daß mehr als 90% der normalen Aldehyde aus der Oxoreaktion zu höheren Aldehyden umgewandelt werden, jedoch der größte Teil des 2-Methylbutanals aus der Oxoreaktion unumgesetzt verbleibt.

3. Verfahren nach einem der Ansprüche 1 und 2, worin die Oxoreaktion mit Propylen- und Butenen-Beschickung und unter derartigen Bedingungen durchgeführt wird, daß Aldehyde mit einem Verhältnis von normalen zu verzweigten Verbindungen von mindestens 2,8:1 hergestellt werden.

4. Verfahren nach einem der Ansprüche 1 bis 3, worin bis zu 20% der Butene Isobutylen ist.

5. Verfahren nach Anspruch 1, worin die Verhältnisse von Propylen und Butenen zusammen mit dem Isobutylengehalt der Butene kontrolliert werden und die Aldolbedingungen so kontrolliert werden, daß Alkohole hergestellt werden, die zumindest zu 70% von normalen Aldehyden abgeleitet sind.

6. Verfahren nach Anspruch 1, worin die Butyl- und Amylaldehyde in einem Verhältnis der

normalen zu den verzweigten Aldehyden von etwa 3:1 vorliegen und das Molverhältnis von Propylen zu Butenen im Bereich von 1:1 bis 1:2 liegt.

7. Verfahren nach Anspruch 1, dadurch modifiziert, daß die Oxoreaktion mit getrennten Propylen- und Butene-Strömen durchgeführt wird, und die Reaktionsprodukte vereint werden.

8. Verfahren nach einem der Ansprüche 1 bis 7, worin mindestens ein wesentlicher Teil des Isobutanals aus der Oxoreaktion von Propylen vom Oxoprodukt durch Destillation vor der Durchführung der Aldolreaktion entfernt wird.

9. Verfahren nach einem der Ansprüche 1 bis 8, worin die Oxoreaktion bei Temperaturen im Bereich von 80° bis 150°C durchgeführt wird und die Drucke hinreichend sind, um die Katalysatorstabilität aufrecht zu erhalten, jedoch nicht oberhalb von 34.500 KPa liegen, und die Aldolreaktion in wässrigem Alkali mit einer Konzentration von 1 bis 10 Gew.-% durchgeführt wird.

10. Verfahren nach einem der Ansprüche 1 bis 9, worin ein wesentlicher Anteil des in dem Verfahren hergestellten Ethylmethylpentanols von den anderen Produktalkoholen durch Destillation abgetrennt wird, um den Produktalkohol als einen Weichmacher-Alkohol zu verbessern.

11. Weichmacher, erhalten aus Weichmacher-Alkoholen, die nach einem Verfahren gemäß einem der Ansprüche 1 bis 10 hergestellt wurden.

**Revendications**

1. Procédé pour la préparation d'alcools pour plastifiants qui consiste à soumettre un mélange d'aldéhydes à une réaction de condensation par aldolisation et à hydrogéner les produits de condensation en alcools saturés, caractérisé en ce que le mélange d'aldéhydes est un mélange de butyl- et d'amyl-aldéhydes obtenu en soumettant un mélange de propylène et de butènes, en un rapport molaire de 2:1 à 1:3, à une réaction oxo.

2. Procédé selon la revendication 1, dans lequel on met en oeuvre la réaction d'aldolisation de manière à transformer plus de 90% des aldéhydes normaux issus de la réaction oxo en aldéhydes supérieurs, mais de manière à ce que la majeure partie du 2-méthylbutanal issu de la réaction oxo ne réagisse pas.

3. Procédé selon l'une ou l'autre des revendications 1 et 2, dans lequel on met en oeuvre la réaction oxo avec une matière première composée de propylène et de butènes et dans des conditions permettant de produire des aldéhydes présentant un rapport des composés normaux aux composés ramifiés d'au moins 2,8:1.

4. Procédé selon l'une quelconque des revendications 1 à 3, dans lequel jusqu'à 20% des butènes sont constitués par l'isobutylène.

5. Procédé selon la revendication 1, dans lequel on règle les proportions de propylène et de butènes ainsi que la teneur en isobutylène des butènes et les conditions de l'aldolisation pour produire des alcools qui sont, au moins pour 70%, dérivés d'aldéhydes normaux.

6. Procédé selon la revendication 1, dans lequel les butyl- et les amylaldéhydes présentent un rapport d'environ 3:1 des aldéhydes normaux aux aldéhydes ramifiés, et le rapport molaire du propylène aux butènes est compris entre 1:1 et 1:2.

7. Procédé selon la revendication 1, modifié dans la mesure où on met en oeuvre la réaction oxo avec des courants distincts de propylène et de butènes, et où on combine les produits de réaction.

8. Procédé selon l'une quelconque des revendications 1 à 7, dans lequel on sépare au moins une partie importante de l'isobutanal issu de la réaction oxo du propylène, du produit de la réaction oxo par distillation avant de mettre en oevure la réaction d'aldolisation.

9. Procédé selon l'une quelconque des revendications 1 à 8, dans lequel on met en oeuvre la réaction oxo à des températures comprises entre 80 et 150°C et sous des pressions suffisantes pour assurer la stabilité, du catalyseur mais ne dépassent pas 34 500 kPa, et dans lequel on met en oeuvre la réaction d'aldolisation dans une base aqueuse à une concentration de 1 à 10% en poids.

10. Procédé selon l'une quelconque des revendications 1 à 9, dans lequel on sépare une partie importante de l'éthylméthylpentanol produit dans le procédé des autres alcools produits par distillation de manière à améliorer les propriétés de l'alcool produit en tant qu'alcool pour plastifiant.

11. Plastifiant obtenu à partir d'alcools pour plastifiants que l'on a préparés selon un procédé conforme à l'une quelconque des revendications 1 à 10.